# EUROPEAN PATENT SPECIFICATION

(11) **EP 3 553 164 B1**
(45) Date of publication and mention of the grant of the patent: **11.05.2022**
(21) Application number: 17878013.6
(22) Date of filing: 24.11.2017
(51) Int. Cl.: C12M 1/34, C12Q 1/06, G01N 21/64, G01N 21/77, G01N 21/78, G01N 33/18

(54) **METHOD FOR INSPECTING MICROORGANISMS, AND APPARATUS FOR SAID METHOD**
VERFAHREN ZUR INSPEKTION VON MIKROORGANISMEN UND VORRICHTUNG FÜR DIESES VERFAHREN
PROCÉDÉ D'INSPECTION DE MICRO-ORGANISMES ET APPAREIL PERMETTANT LA MISE EN OEUVRE DUDIT PROCÉDÉ

(30) Priority: 09.12.2016 JP 2016239302
(43) Date of publication of application: 16.10.2019
(73) Proprietor: Satake Corporation, Chiyoda-ku Tokyo 101-0021 (JP)
(72) Inventor: HE Jianjun, Tokyo 101-0021 (JP); MATSUDA Masanori, Tokyo 101-0021 (JP)
(74) Representative: Haseltine Lake Kempner LLP
(86) International application number: PCT/JP2017/042274
(87) International publication number: WO 2018/105414

(56) References cited:
- WO-A1-2006/103932
- WO-A1-2006/103932
- CN-A- 103 868 901
- JP-A- H0 815 157
- JP-A- H08 242 886
- JP-A- H08 242 886
- JP-A- 2001 083 094
- JP-A- 2006 284 335
- JP-A- 2013 050 375
- JP-A- 2013 050 375
- JP-A- 2014 042 463
- JP-A- 2014 042 463
- JP-A- 2014 055 796
- JP-A- 2014 055 796
- KR-A- 20170 038 427
- PEPERZAK, L. ET AL.: 'Flow cytometric applicability of fluorescent vitality probes on phytoplankton' JOURNAL OF PHYCOLOGY vol. 47, 09 May 2011, ISSN 1529-8817 pages 692 - 702, XP055324703
- BAEK, S. H. ET AL.: 'Applicability of fluorescein diacetate (FDA) and calcein- AM to determine the viability of marine plankton' OCEAN AND POLAR RESEARCH vol. 31, no. 4, 30 December 2009, ISSN 1598-141X pages 349 - 357, XP055491457

## Description

### [Technical Field]

The present invention relates to a microorganism testing method and an apparatus for the method and, in particular, to a microorganism testing method suitable for detecting microorganisms such as living plankton included in ballast water or the like, and an apparatus for the method.

### [Background Art]

A ship without cargo sails loaded with ballast water in order to stabilize the ship and discharges the ballast water in a sea area where cargo is loaded.

Ballast water is usually discharged in a sea area different from a sea area where the ballast water is loaded. Therefore, there is a possibility that microorganisms such as plankton and bacteria included in the ballast water are carried to an area other than an original habitat, and problems of destruction of an ecosystem and the like are caused.

In order to cope with such problems, international rules about regulations of ballast water were formulated, and "International Convention for the Control and Management of Ships' Ballast Water and Sediments (the Ballast Water Management Convention)" has been adopted.

In "Guidelines for ballast water sampling (G2)" related to the Ballast Water Management Convention, permissible populations of living organisms included in ballast water discharged from ships are stipulated by classifying the permissible populations based on minimum sizes of the organisms, for example, 10 organisms/m³ or fewer for organisms the minimum size of which is 50 µm or larger (hereinafter referred to as "L-size organisms"), 10 organisms/mL or fewer for organisms the minimum size of which is between 10 µm and 50 µm, including 10 µm and excluding 50 µm (hereinafter referred to as "S-size organisms) and the like in "the ballast water discharge standard (D-2)".

As techniques for confirming whether the discharge standard is satisfied or not at the time of discharging the ballast water, a technique in which seawater is pumped through a flow cell to measure an image (for example, Patent Literature 1), an apparatus collecting seawater as sample water after flowing seawater pumped by a pump through filters with different openings, adding a staining reagent to the sample water and irradiating excitation light while stirring the sample water, detecting light of fluorescence emission caused by the excitation light and counting the number of light emissions, and calculating the number of microorganisms included in the sample water from the number of light emissions (for example, Patent Literature 2 and Patent Literature 3) and the like have been known up to now.

The apparatus described in Patent Literature 1 is provided with a staining portion staining organisms with live cells existing in a liquid specimen while flowing the liquid specimen, a concentrating portion concentrating the stained specimen so that concentration of the organisms is increased while flowing the stained specimen, an individual measuring portion acquiring image information about individuals including the organisms in the concentrated specimen, and a control means performing measurement of the organisms from the image information about the individuals outputted from the individual measuring portion.

Thereby, a process of staining organisms in specimen liquid, a process of concentrating the organisms in the liquid, a process of acquiring information about the organisms in the liquid and the like can be performed by a flow method. Therefore, in comparison with a technique of performing each process by batch, it is possible to significantly shorten or eliminate waiting time required until a part of the specimen for which one process has been finished proceeds to the next process. Thus, there is an advantage of acquiring stable information about life or death of organisms in the sense of preventing deterioration of a stained state during the waiting time.

In the above apparatus according to Patent Literature 1, however, seawater is pumped sequentially through various kinds of processes, and there is a problem that the apparatus is large-scaled, and the manufacturing cost increases. Moreover, though water is pumped sequentially through the various kinds of processes so that the waiting time is shortened, there is a problem that at least several hours are required to complete measurement.

Each of the apparatuses described in Patent Literature 2 and 3 is provided with: stirring/mixing means performing stirring/mixing of sample solution obtained by adding a sample and a fluorescent staining reagent into a batch-type sample container formed of material transmitting light, an excitation light source provided with light sources irradiating excitation light to an irradiated surface of the sample container while the sample solution is being stirred by the stirring/mixing means, photodetector detecting light of fluorescence emission caused by the excitation light from the excitation light source, and control means converting the light detected by the photodetector to an electrical signal to detect the number of light emissions and calculating the number of microorganisms included in the sample within the sample container from the number of light emissions.

Thereby, after adding a sample and a fluorescent staining reagent into a batch-type sample container, stirring/mixing in the sample container is performed by the stirring/mixing means; excitation light is then caused to be incident to the irradiated surface of the sample container while the sample solution is being stirred, and; furthermore, fluorescence emission of microorganisms is received by the photodetector. Therefore, in comparison with the case of performing measurement with leaving sample solution standing without stirring, the microorganisms brightly emit light in an extremely short time, and it becomes possible to easily measure the number of microorganisms in ballast water in a short time. Because of the batch type, it becomes possible to downsize the apparatus, and there is an advantage that the manufacturing cost decreases.

In the above apparatuses described in Patent Literature 2 and 3, however, there is a problem that it is difficult to detect some phytoplankton. Especially as for some diatoms having siliceous (glassy) shells around cells, among algae, which are phytoplankton, a staining agent FDA (a fluorescent staining reagent FDA) is not easily taken in, and, therefore, the amount of fluorescence emission is small, and detection is difficult.

### [Citation List]

### [Patent Literature]

[Patent Literature 1]
   Japanese Patent Laid-Open No. 2009-85898
[Patent Literature 2]
   Japanese Patent Laid-Open No. 2014-42463
[Patent Literature 3]
   Japanese Patent Laid-Open No. 2014-55796

### [Summary of Invention]

### [Technical Problem]

In view of the above problems, the technical subject of the present invention is to provide a method for detecting microorganisms in ballast water, the method capable of easily detecting such phytoplankton that a fluorescent staining reagent is not easily taken in, in a short time, and an apparatus for the method.

### [Solution to Problem]

In order to solve the above subject, a microorganism testing apparatus according to the present invention is set out in the appended set of claims

According to the microorganism testing apparatus of the present invention, since the two different kinds of excitation light sources, i.e. the light source emitting the light with the wavelength region causing phytoplankton to emit chlorophyll fluorescence and the light source emitting the light with the wavelength region causing microorganisms stained by the fluorescent staining reagent to emit fluorescence are used for the excitation light source, it becomes possible to detect such phytoplankton that the fluorescent staining reagent is not easily taken in, by using the light source emitting the light with the wavelength region causing chlorophyll fluorescence emission, and, thereby, it becomes possible to easily detect both of phytoplankton and zooplankton without any omission in a short time.

In the microorganism testing apparatus, the excitation light source is arranged such that excitation light emitted therefrom is incident orthogonally to the irradiated surface of the sample container, and a light receiving surface of the photodetector is arranged such that fluorescence emission is received at an angle orthogonal to the excitation light of the excitation light source.

According to the microorganism testing apparatus, the excitation light source is arranged such that the excitation light emitted therefrom is incident orthogonally to the irradiated surface of the sample container, and the light receiving surface of the photodetector is arranged such that fluorescence emission is received at the angle orthogonal to the excitation light of the excitation light source. Therefore, the excitation light from the excitation light source is not incident directly to the light receiving surface of the photodetector, and difference in the amount of light between a background and fluorescence emission of microorganisms becomes extremely clear. Thus, the microorganism detection accuracy is improved.

In the microorganism testing apparatus, the control means is provided with an operation unit calculating a permissible number of microorganisms N with respect to a ballast water discharge criterion after determining each of a microbial population n1 acquired by chlorophyll fluorescence emission, a microbial population n2 acquired by fluorescence emission by the fluorescent staining reagent and a microbial population n3 acquired by both of the chlorophyll fluorescence emission and the fluorescence emission by the fluorescent staining reagent.

According to the above microorganism testing apparatus, the control means estimates the microbial population n3 as the permissible microbial population N for a complemented number after determining each of the microbial population n1 acquired by chlorophyll fluorescence emission, the microbial population n2 acquired by fluorescence emission by the fluorescent staining reagent and the microbial population n3 acquired by both of the chlorophyll fluorescence emission and the fluorescence emission by the fluorescent staining reagent. The permissible population N makes it possible to appropriately evaluate the number of microorganisms and evaluate and apply the ballast water discharge standards (D-2) the same as true evaluation.

A microorganism testing method according to the present invention is set out in the appended set of claims.

In the microorganism testing method, the number-of-microorganisms estimating process calculates a permissible number of microorganisms N with respect to a ballast water discharge criterion after determining each of a microbial population n1 acquired by chlorophyll fluorescence emission, a microbial population n2 acquired by fluorescence emission by the fluorescent staining reagent and a microbial population n3 acquired by both of the chlorophyll fluorescence emission and the fluorescence emission by the fluorescent staining reagent.

According to the above microorganism testing method, a method for detecting such phytoplankton that a fluorescent staining reagent is not easily taken in by using the light source emitting light with the wavelength region causing chlorophyll fluorescence emission is realized. Thereby, it is possible to easily detect both of phytoplankton and zooplankton without any omission in a short time.

In the microorganism testing method, the microorganism estimating process calculates a population of zooplankton by subtracting the microbial population n2 acquired by the fluorescence emission by the fluorescent staining reagent from the microbial population n3 acquired by both of the chlorophyll fluorescence emission and the fluorescence emission by the fluorescent staining reagent.

According to the microorganism testing method, it becomes possible to, by subtracting the microbial population n2 acquired by the fluorescence emission by the fluorescent staining reagent from the microbial population n3 acquired by both of the chlorophyll fluorescence emission and the fluorescence emission by the fluorescent staining reagent, calculate and count only the population of zooplankton by the microorganism estimating process.

### [Advantageous Effects of Invention]

According to the present invention, it is possible to provide a method for detecting microorganisms in ballast water, the method capable of easily detecting such phytoplankton that a fluorescent staining reagent is not easily taken in, in a short time, and an apparatus for the method.

### [Brief Description of Drawings]

[Figure 1] Figure 1 is a perspective view showing a whole microorganism testing apparatus.
[Figure 2] Figure 2 is a schematic cross-sectional plan view of a measuring portion of the microorganism testing apparatus.
[Figure 3] Figure 3 is a block diagram showing an overall configuration of the measuring portion.
[Figure 4] Figure 4 is a schematic cross-sectional plan view of a measuring portion capable of easily detecting such phytoplankton that a fluorescent staining reagent is not easily taken in.
[Figure 5A] Figure 5A is a schematic cross-sectional view showing one embodiment of collimator.
[Figure 5B] Figure 5B is a schematic cross-sectional view showing another example of the one embodiment of the collimator.
[Figure 6A] Figure 6A is a diagram showing a field of view obtained without a slit.
[Figure 6B] Figure 6B is a diagram showing the field of view narrowed with a slit.
[Figure 7] Figure 7 is a flowchart showing a measurement flow of the microorganism testing apparatus.
[Figure 8A] Figure 8A is a Venn diagram of sets related to microbial populations n1 and n2.
[Figure 8B] Figure 8B is a Venn diagram of a set related to a microbial population n3 at the time of simultaneously radiating the two kinds of LED light sources.
[Figure 8C] Figure 8C is a Venn diagram of a set obtained by subtracting the microbial population n2 detected by the fluorescent staining reagent from the microbial population n3 at the time of simultaneously radiating the two kinds of LED light sources.
[Figure 9] Figure 9 is a schematic plan view showing a modification 1 of the measuring portion of Figure 4.
[Figure 10] Figure 10 is a schematic plan view showing a modification 2 of the measuring portion of Figure 4.
[Figure 11] Figure 11 is a schematic plan view showing a third modification 3 of the measuring portion of Figure 4.
[Figure 12] Figure 12 is a graph showing a test of whether such phytoplankton that the fluorescent staining reagent is not easily taken in can be detected or not.
[Figure 13] Figure 13 is a graph showing the second test of the above.
[Figure 14] Figure 14 is an explanatory diagram when received signals of photodetectors 9A and 9B of the modification 2 are compared.

### [Description of Embodiment]

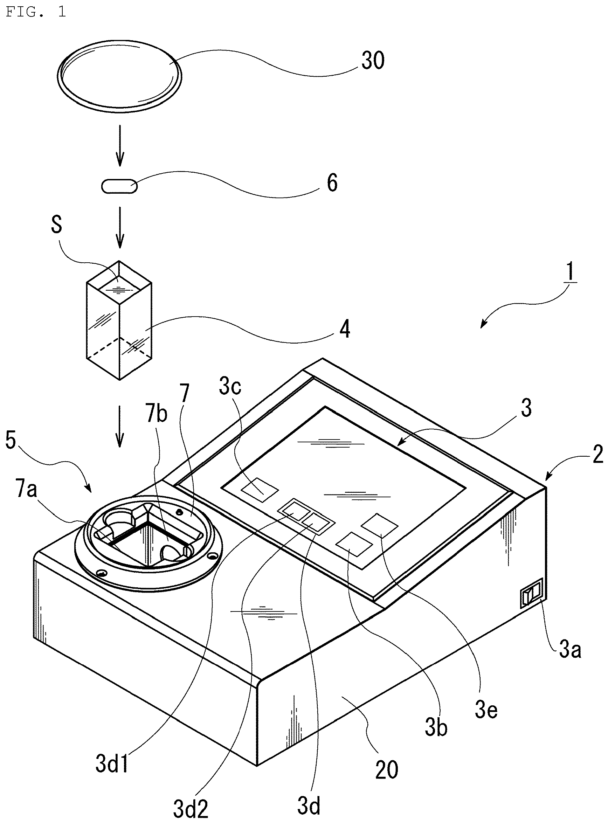

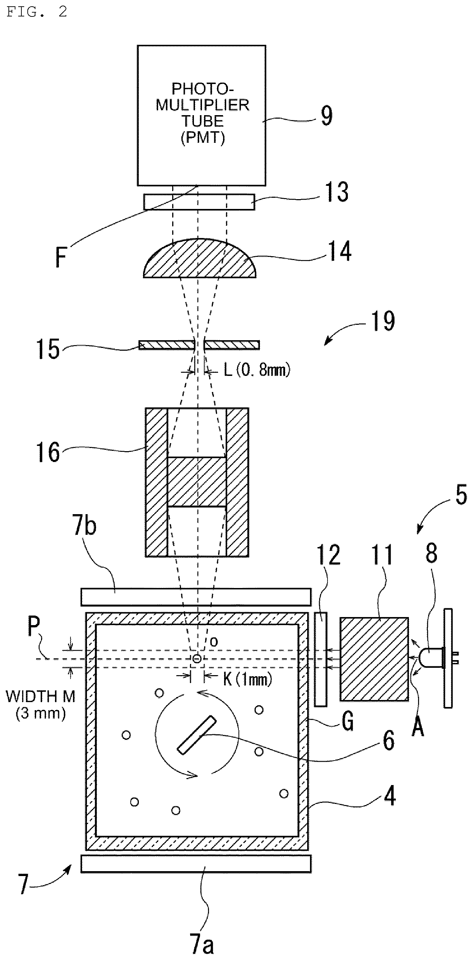

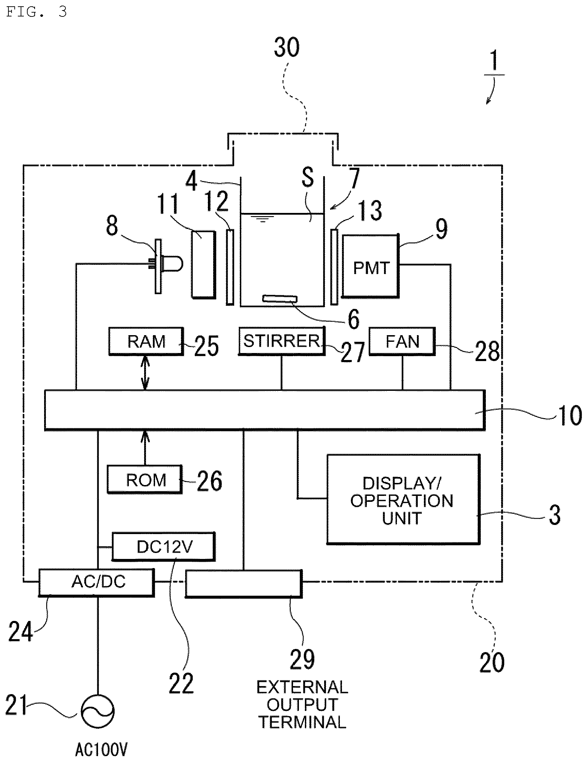

An embodiment for practicing the present invention will be described with reference to drawings. Figure 1 is a perspective view showing a whole microorganism testing apparatus; Figure 2 is a schematic cross-sectional plan view of a measuring portion of the microorganism testing apparatus; and Figure 3 is a block diagram showing an overall configuration of the measuring portion.

As shown in Figures 1, 2 and 3, the testing apparatus is configured with a body portion 2 that includes a control apparatus such as a CPU board and performs information processing work, statistical processing work and the like for measurement results and the like, a display/operation unit 3 on which operation icons and the like corresponding to operation buttons and the like that respond to a touch on a screen by a finger are arranged, and which is formed by a liquid crystal touch panel for displaying the measurement results and the like, and a measuring portion 5 that accommodates a batch-type sample container 4 formed of transparent material that transmits light (for example, glass, quarts, acrylic resin or the like) and that optically counts the number of microorganisms in sample solution S, the display/operation unit 3 and the measuring portion 5 being provided on the body portion 2 in line, as main portions.

Reference numeral 6 indicates a stirrer bar for stirring the sample solution S accommodated in the sample container 4. In the sample container 4, a sample, a luminescent reagent (a combination of the sample and the luminescent reagent is assumed to be the sample solution S) and the stirrer bar 6 are accommodated. A configuration is provided in which, when the sample container 4 is accommodated in the measuring portion 5, the stirrer bar 6 is driven and rotated by a magnetic stirrer built in the measuring portion 5. Thereby, it is possible to count the number of microorganisms in the sample solution S while stirring and mixing the sample solution S in the sample container 4 at a predetermined temperature. That is, in comparison with the case of counting the number of microorganisms in the sample solution S left standing, microorganisms brightly emit light in an extremely short time, and it becomes possible to easily measure the number of microorganisms in ballast water in a short time.

Dimensions of the testing apparatus 1 shown in Figure 1 are: width=300 mm, depth=350 mm and height=130 mm. The weight is within a range of about 2 to 5 kg. The testing apparatus 1 can be accommodated in a handheld suitcase, a rucksack (also referred to as "a backpack") (neither of them is shown) or the like and can be carried anywhere. The testing apparatus 1 is designed to be also driven by an AC power source or a battery so that measurement in a ship and outdoors is possible.

The sample container 4 is formed of transparent material that transmits light and is formed in a prismatic shape with a bottom face of 50 mm×50 mm and a height of 60 mm. The amount of content when a water level is 40 mm is set to 100 ml (milliliters). The sample container 4 is not limited to such a prismatic shape but may be in a cylindrical shape or a cubic shape if the amount of content of about 100 ml (milliliters) can be secured.

As shown in Figures 1, 2 and 3, the measuring portion 5 is provided with a sample container accommodating portion 7 that accommodates and holds the sample container 4, a light source portion 8 that radiates excitation light toward the sample container 4, and a photodetector 9 for observing microorganisms drifting in the sample container 4 through the excitation light irradiated from the light source portion 8. From the photodetector 9, communication is electrically performed to a CPU board 10 that counts the number of microorganisms in the sample solution S and performs information processing work, statistical processing work and the like of a measurement result and the like.

The sample container accommodating portion 7 is formed by holding plates 7a and 7b surrounding at least two faces of the sample container 4, and accommodates and holds the sample container 4 such that radiation of light from the light source portion 8 is not blocked.

As shown in Figure 2, the light source portion 8 is arranged such that excitation light along a normal line AP is incident to an irradiated surface G of the sample container 4. The light source portion 8 is provided with an LED light source 8 arranged near the sample container accommodating portion 7, collimator 11 arranged on the front of the LED light source 8, the collimator 11 converting diffused light to parallel light (since an LED device emits light that is diffusedly radiated in random directions from a device side, the light is to be converted to parallel light to apply light beams uniformly to one surface at the same angle), and a band pass filter for excitation light 12 that causes excitation light constituted by the parallel light to be radiated to the sample container 4.

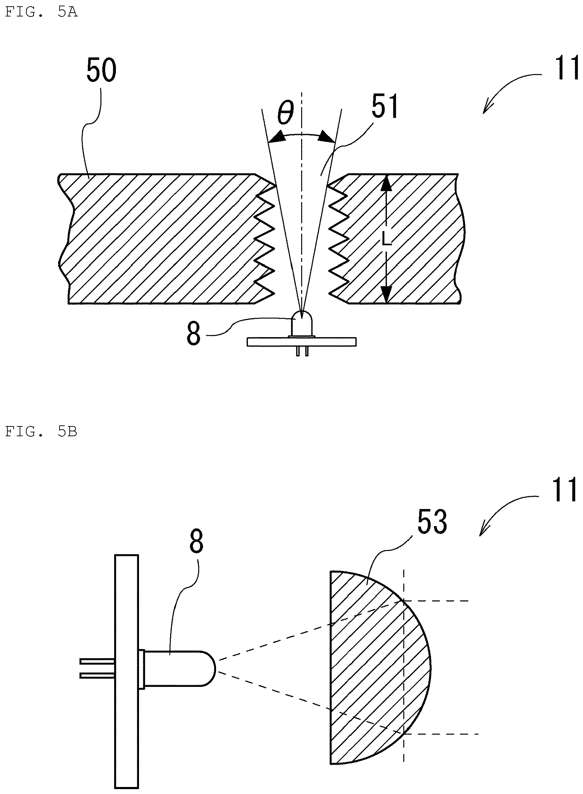

Figure 5 shows schematic cross-sectional views showing one embodiment of the collimator 11. An example shown in Figure 5A is an example in which the collimator 11 is formed by drilling a threaded hole 51 of a predetermined diameter in a flat plate 50 of a predetermined thickness, and a thickness L of the flat plate 50 and a hole diameter of the threaded hole 51 are appropriately set according to an optical path length. Thereby, diffused light at an incident angle θ that is irradiated from the LED light source 8 is converted to parallel light when passing through the threaded hole 51. In an example shown in Figure 5B, an optimal condition between θ and L is decided by an S/N ratio test. For example, when M3 (screw hole outer diameter)×0.5 (pitch) is assumed, an optimal result is obtained when θ is 9.5°, and L is 15 mm.

The collimator 11 shown in Figure 5B is provided with a convex lens 53 on the front of the LED light source 8, and diffused light radiated from the LED light source 8 is converted to parallel light when passing through the convex lens 53 to be emitted outside.

Though the light source portion 8 of the present embodiment uses an LED light source as a light source, the light source portion 8 is not limited to an LED light source, but a parallel light LED light source, a laser light source or a light bulb capable of radiating parallel light can be adopted if it is possible to cause fluorescent materials included in microorganisms to be excited. It goes without saying that, in the case of adopting a parallel light LED, a laser light source or a light bulb capable of radiating parallel light, the collimator 11 described above is unnecessary.

As shown in Figure 2, the photodetector 9 is provided such that a light receiving surface F is arranged at an angle orthogonal to excitation light along the normal line AP from the light source portion 8. The photodetector 9 is also provided with a photomultiplier tube (PMT) 9 arranged and configured so that fluorescence is received along a light axis orthogonal to such parallel light that excitation light is radiated from the LED light source 8 toward the sample container 4, a band pass filter for fluorescence 13 arranged on the front of the photomultiplier tube (PMT) 9, a condenser lens 14 arranged on the front of the band pass filter for fluorescence 13, a slit 15 arranged on the front of the condenser 14 and a relay lens 16 installed in a gap between the slit 15 and the sample container 4, the relay lens 16 being for causing fluorescent materials included in microorganisms to excite and condensing fluorescence emitted thereby to form an image.

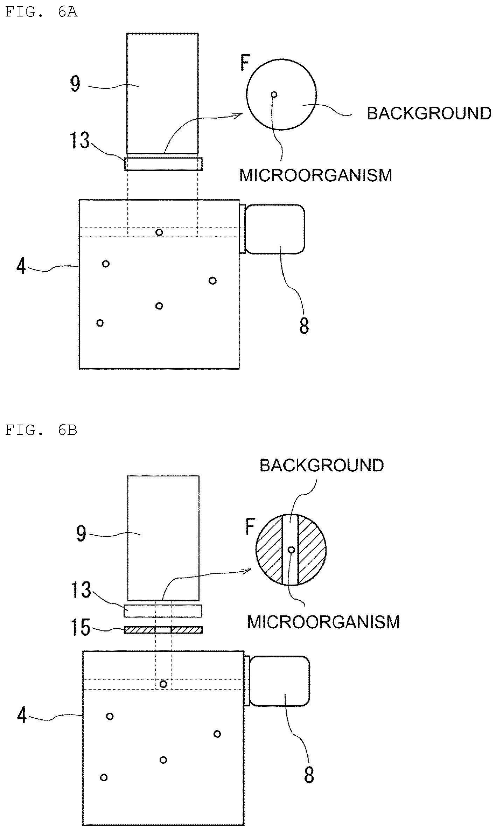

The slit 15 narrows a field of view to be in a slit shape. That is, as shown in Figure 6, though such a background that the light receiving surface F is formed in a circle is monitored in a state without a slit in Figure 6A, such a background that the light receiving surface F is formed by a rectangular slit excluding shaded parts is monitored in a state with a slit in Figure 6B. Therefore, as a result of a monitoring area (a monitoring range) on the observation surface F being narrowed as in Figure 6B, the area of fluorescence emission on the background, which is to be noise, is also narrowed. Thus, the ratio of a signal of fluorescence emission of microorganisms to fluorescence emission of the background is improved, and the accuracy of detection of the fluorescence emission of the microorganisms is improved.

Though an example has been shown in which the photodetector 9 uses a photomultiplier tube (PMT) as a photodetector, the photodetector is not limited to a photomultiplier tube (PMT), but various kinds of light detectors capable of detecting light emission of fluorescent materials included in microorganisms similarly to a photomultiplier tube (PMT), such as a silicon photodiode (SiPD) and an avalanche photodiode (APD), can be adopted.

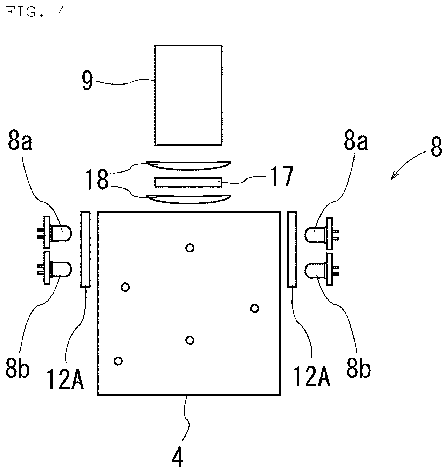

Next, description will be made on a configuration capable of easily detecting such phytoplankton that a fluorescent staining reagent is not easily taken in, the configuration being a main part of the present invention, with reference to Figure 4.

The light source portion 8 shown in Figure 4 is characterized in that two kinds of LED light sources 8a, 8b with different wavelength regions are used. That is, the LED light source 8 is provided with a pair of the LED light source 8a that emits bluish-green light around a wavelength region of 490 nm (a light source similar to a conventional one) and the LED light source 8b that emits bluish-purple light around a wavelength region of 450 nm. It is recommended to provide the pair of the LED light sources 8a, 8b in such a manner of facing each other sandwiching the sample container 4. Between the LED light sources 8a, 8b and the sample container 4, band pass filters for excitation light 12A, 12A that transmit light with a wavelength region of 395 to 505 nm are interposed, respectively, so that light on the LED light source 8b side is easily transmitted. These wavelength regions are merely an example and can be appropriately changed according to conditions.

A long pass filter 17 that transmits a light with a wavelength region of 510 nm or more is provided on the front of the photodetector 9 shown in Figure 4. Furthermore, condenser lenses 18 are arranged in front of and behind the long pass filter 17 to sandwich the long pass filter 17.

Furthermore, an electrical control configuration will be described with reference to Figure 3. In the center inside a case 20 forming the body portion 2, the CPU board 10 is arranged, the CPU board 10 receiving power source supply from an AC power source 21 or a secondary battery 22 to analyze an output signal converted from light to electricity by the photomultiplier tube (PMT) 9, judge whether or not brightness is within or above an arbitrary brightness range, count pulses of a signal with an arbitrary brightness and perform on/off control of the LED light source 8. An AC/DC converter 24 is interposed between the AC power source 21 and the CPU board 10.

Each of the photomultiplier tube (PMT) 9, the LED light source 8, a RAM 25 to be a storage portion for reading and writing and a ROM 26 to be a storage portion dedicated for reading is electrically connected to the CPU board 10. Further, they are electrically connected to the display/operation unit 3 formed by a liquid crystal touch panel or the like shown in Figure 1. A configuration is provided in which on/off switching control is performed by pressing down a power source button 3a displayed on the liquid crystal panel, measurement is started by pressing down a measurement start button 3b, transfer of data to an external printer or personal computer is performed by pressing down an external output button 3c, switching between measurement types (switching between measurement of L-size microorganisms (3d1) or measurement of S-size microorganisms (3d2)) is performed by pressing down a setting button 3d, and change of setting of a judgment criterion, change of setting of a threshold and change of setting of measurement time can be performed by pressing down a menu button 3e, as described later.

In addition, a magnetic stirrer 27 that causes the stirrer bar 6 to rotate by magnetic force, a cooling fan 28 for control equipment, and external output terminals 29, such as RS-232C and universal serial bus (USB) terminals, are connected to the CPU board 10.

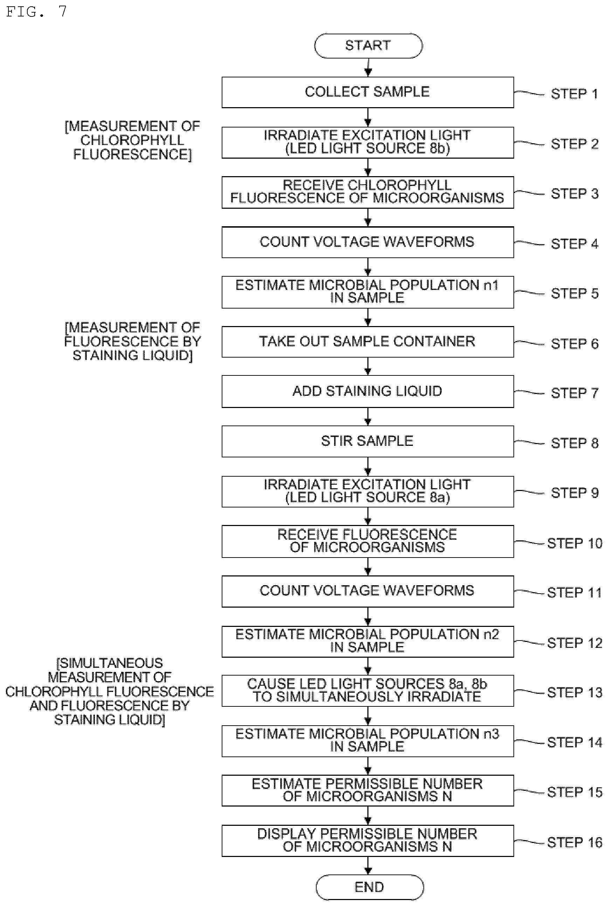

Figure 7 is a flowchart showing a measurement flow. Operation in the above configuration will be described with reference to Figures 1 to 7.

### [Measurement of chlorophyll fluorescence]

First, measurement of chlorophyll fluorescence is started. A operator takes 100 ml (milliliters) of ballast water as a sample using a pipette or the like and injects the ballast water into the sample container 4 (step 1 in Figure 7). Next, by accommodating the sample container 4 into the measuring portion 5 of the testing apparatus 1 and applying a cover 30 of the measuring portion 5, measurement preparation is completed.

The operator turns on the power source button 3a on the body portion 2 and makes preparations by pressing down the setting button 3d, the menu button 3e and the like on the display/operation unit 3 configured with a liquid crystal touch panel. After that, the measurement start button 3b is turned on. Thereby, the LED light sources 8b, 8b for chlorophyll fluorescence are lit up (see Figure 4 and step 2 in Figure 7), and light transmitted through the band pass filters for excitation light 12A, 12A (Figure 4) is irradiated to the sample container 4. At this time, for example, light with a wavelength of 450 nm as a wavelength characteristic is irradiated, and chlorophyll components of a specimen (microorganisms) in the sample container 4 emit fluorescence. The fluorescence by the chlorophyll components is transmitted through the long pass filter 17 and detected by the photomultiplier tube (PMT) 9 (step 3 in Figure 7).

In the photomultiplier tube (PMT) 9, light energy is converted to electrical energy by using a photoelectric effect, and a current amplifying function is added, so that fluorescence emission of the chlorophyll components with a high sensitivity. A detected electrical signal is sent to the CPU board 10, and received light waveforms at or above a predetermined threshold are counted (step 4 in Figure 7).

Furthermore, in the CPU board 10, the number of microorganisms existing in the 100 ml (milliliters) of water in the sample container 4 is estimated from the counted value of the received light waveforms, and the number of microorganisms is displayed on the display/operation unit 3 (step 5 in Figure 7).

### [Example 1]

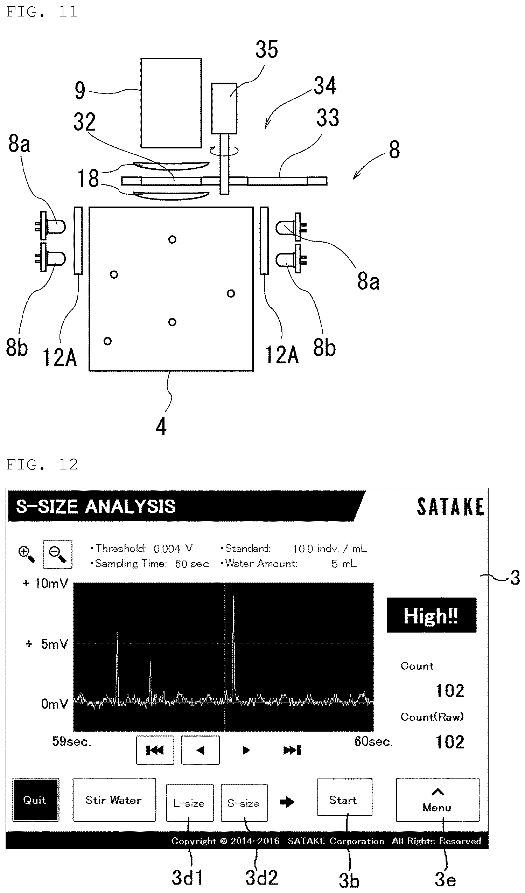

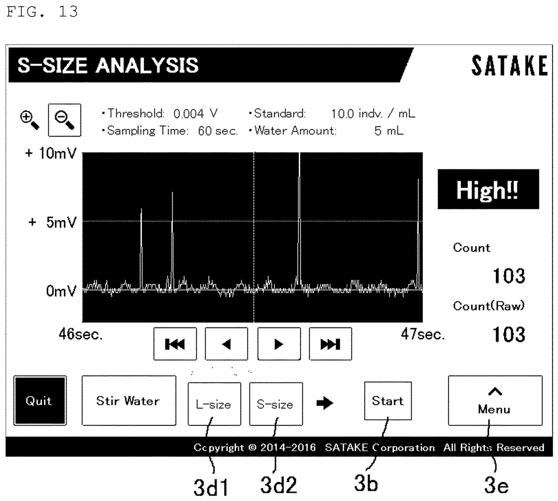

With Prorocentrum micans, which is a kind of phytoplankton, used as test microorganisms, it was verified whether or not the population can be estimated by the photomultiplier tube (PMT) 9 by chlorophyll fluorescence. A plurality of Prorocentrum micans individuals are accommodated in the sample container 4 (with a capacity of 100 mL) together with water, and the counted number of waveforms was detected (see Figures 12 and 13). As a result, from the obtained counted number of waveforms, a population of 102 was counted in Figure 12, and a population of 103 was counted in Figure 13. That is, it was known that, for phytoplankton existing in the 100 mL of ballast water, especially even for phytoplankton that does not easily absorb FDA and the like, a microbial population can be estimated by chlorophyll fluorescence emission without absorption of FDA. In the CPU board 10, the microbial population at this time is stored as n1 (step 5 in Figure 7).

### [Measurement of fluorescence by staining liquid]

Next, returning to Figure 7, measurement of fluorescence by staining liquid will be described. The sample container 4 after the measurement of the chlorophyll fluorescence emission is taken out of the testing apparatus 1 (step 6 in Figure 7), and a fluorescent staining reagent is added into the taken-out sample container 4 (step 7 in Figure 7).

Commonly known Calcein-AM (manufactured by PromoCell GMBH in Germany), FDA or the like can be used as the fluorescent staining reagent. Calcein-AM tends to stain phytoplankton, while FDA tends to stain zooplankton. Then, by the operator causing the sample container 4 to be accommodated in the measuring portion 5 of the testing apparatus 1 after putting the stirrer bar 6 into the sample container 4 and applying the cover 30, measurement preparation is completed.

Here, the operator presses down an S size setting button 3d2 (or L size 3dl) on the display/operation unit 3 and turns on the measurement start button 3b. Then, the stirrer bar 6 rotates by driving of the magnetic stirrer 27 built in the measuring portion 5, and the sample solution S is stirred (step 8 in Figure 7).

Next, the LED light sources 8a, 8a are lit up (see Figure 4), and light transmitted through the band pass filters for excitation light 12A, 12A is irradiated to the sample container 4. At this time, for example, light around a wavelength region of 490 nm as a wavelength characteristic is irradiated, and the specimen (the microorganisms) in the sample container 4 emit fluorescence. Then, the fluorescence is transmitted through the band pass filter for fluorescence 15 and detected by the photomultiplier tube (PMT) 9 (step 10 in Figure 7).

A detected electrical signal detected by the photomultiplier tube (PMT) 9 is sent to the CPU board 10, and received light waveforms at or above a predetermined threshold are counted (step 11 in Figure 7). Furthermore, in the CPU board 10, the number of microorganisms existing in the 100 ml (milliliters) of water in the sample container 4 is estimated from the counted value of the received light waveforms, and the number of microorganisms is displayed on the display/operation unit 3. In the CPU board 10, the microbial population at this time is stored as n2 (step 12 in Figure 7).

### [Measurement of both of chlorophyll fluorescence and fluorescence by staining liquid]

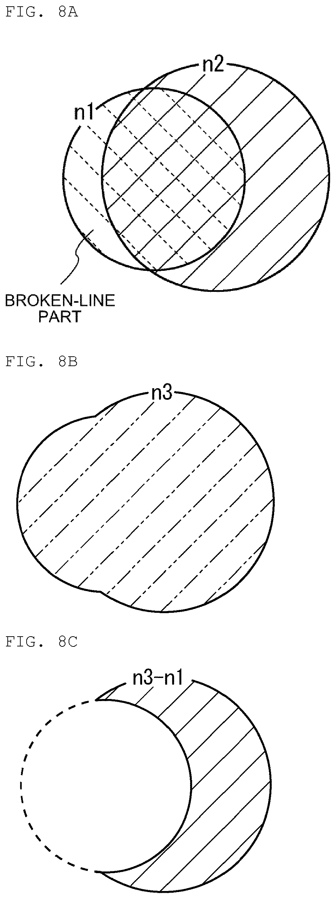

Then, both of the LED light sources 8a, 8a and the LED light sources 8b, 8b are caused to simultaneously radiate (step 13 in Figure 7); detection is performed by the photomultiplier tube (PMT) 9; and the microbial population at this time is stored as n3 (step 14 in Figure 7). Here, description will be made on a relationship among the microbial population n1 detected by the chlorophyll fluorescence emission, the microbial population n2 detected by the fluorescence emission using the fluorescent staining reagent and the microbial population n3 at the time of simultaneously radiating the two kinds of LED light sources 8a, 8b, using Figure 8.

Figure 8 shows Venn diagrams of sets related to the microbial populations n1, n2 and n3 acquired by the above process. Figure 8A shows a logical sum set obtained by two sets of the microbial population n1 and the microbial population n2 being combined. Since only the microbial population n2 detected by fluorescence emission using a fluorescent staining reagent has been evaluated heretofore, there is a possibility that the population of phytoplankton that does not easily absorb the fluorescent staining reagent (a broken-line part of the microbial population n1), the phytoplankton not having been detected conventionally, has not been taken into account. This means that an allowable microbial population is estimated less than the true allowable microbial population, and the ballast water discharge standard (D-2) is also evaluated more loosely than true evaluation.

Therefore, if the microbial population at the time of simultaneously radiating the two kinds of LED light sources 8a, 8b is assumed as n3 as in Figure 8B, the population of phytoplankton that does not easily absorb the fluorescent staining reagent is added, and an appropriate permissible microbial population can be obtained. The CPU board 10 estimates the microbial population n3 at the time of simultaneously radiating the two kinds of LED light sources 8a, 8b as a permissible microbial population N for a complemented number (step 15 in Figure 7), and the permissible population N is displayed on the display/operation unit 3 based thereon (step 16 in Figure 7). Since the permissible population N appropriately estimates the number of microorganisms, it is possible to evaluate and apply the ballast water discharge standards (D-2) the same as true evaluation.

Figure 8C is a diagram in which the microbial population n1 detected by the chlorophyll fluorescence emission is subtracted from the microbial population n3 at the time of simultaneously radiating the two kinds of LED light sources 8a, 8b. The set of n3-n1 is such that the set n1 of only phytoplankton is subtracted from the set n3 in which zooplankton and the phytoplankton coexist, and only the population of the zooplankton can be determined.

The [measurement of chlorophyll fluorescence] described in paragraph 0042 and the [measurement of fluorescence by staining liquid] described in paragraph 0047 may be exchanged in order and implemented. Further, the [measurement of both of chlorophyll fluorescence and fluorescence by staining liquid] described in paragraph 0052 may be implemented first.

As described above, according to the present embodiment, there is provided a microorganism testing apparatus provided with the body portion 2, the display/operation unit 3 and the measuring portion 5 optically counting the number of microorganisms in the sample solution S accommodated in the batch-type sample container 4, the display/operation unit 3 and the measuring portion 5 being arranged in line on the body portion 2, wherein
the measuring portion 5 is configured being provided with the sample container accommodating portion 7 accommodating and holding the sample container 4, the light source portion 8 radiating excitation light toward the sample container 4, and the photodetector 9 for observing microorganisms drifting in the sample container 4 by the excitation light radiated from the light source portion 8; and
the two different kinds of LED light sources 8a, 8b with different wavelength regions (especially, the LED light source 8a emitting bluish-green light around the wavelength region of 490 nm (a light source similar to a conventional one) and the LED light source 8b emitting bluish-purple light around the wavelength region of 450 nm are provided as a pair) are used for the light source portion 8. Therefore, by easily detecting such phytoplankton that a fluorescent staining reagent is not easily taken in, in a short time, it becomes possible to detect both of zooplankton and phytoplankton without failure.

### [Example 2]

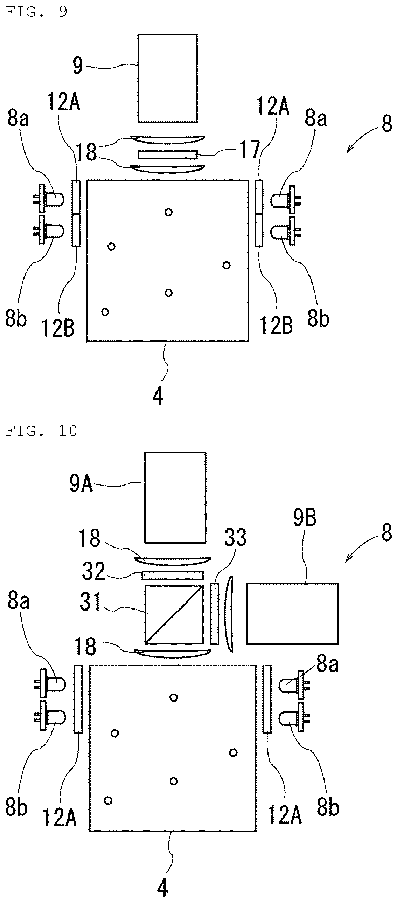

Figure 9 shows a modification 1 of the measuring portion, the modification 1 being characterized in that the two kinds of LED light sources 8a, 8b are provided with dedicated band pass filters 12A, 12B, respectively, in comparison with a basic example of the measuring portion of Figure 4.

Figure 10 shows a modification 2 of the measuring portion, the modification 2 being characterized in that a dichroic mirror 31 capable of spectroscopy and two photodetectors 9A, 9B with sensitivities specific to a wavelength obtained by spectroscopy are provided, in comparison with the basic example of the measuring portion of Figure 4. A long pass filter 33 that transmits a wavelength region with a 650 nm or more is interposed between the photodetector 9A and the dichroic mirror 31, and a band pass filter 32 with a wavelength region of 510 to 550 nm is interposed between the photodetector 9A and the dichroic mirror 31.

Figure 11 shows a modification 3 of the measuring portion, the modification 3 being characterized in that, instead of the single long pass filter 17, a filter wheel 34 configured with the band pass filter 32 with the wavelength region of 510 to 550 nm and the long pass filter 33 that transmits the wavelength region of 650 nm or more is arranged, in comparison with the basic example in Figure 4. Reference numeral 35 in Figure 11 indicates a step motor that drives the filter wheel 34.

In the modification 1 of the measuring portion shown in Figure 9, the modification 2 of the measuring portion shown in Figure 10 and the modification 3 of the measuring portion shown in Figure 11, the light source portion 8 is provided with the two kinds of LED light sources 8a, 8b with different wavelength regions, and a filter and a photodetector specific to each of the light sources are provided. Therefore, by easily detecting such phytoplankton that a fluorescent staining reagent is not easily taken in, in a short time, both of zooplankton and phytoplankton can be detected without failure.

Further, as described before, the CPU board 10 determines each of the microbial population n1 acquired by chlorophyll fluorescence emission, the microbial population n2 acquired by fluorescence emission by staining liquid and the microbial population n3 acquired by both of the chlorophyll fluorescence emission and the fluorescence emission by the staining liquid.

The microbial population n3 is such that the population of phytoplankton that does not easily absorb a fluorescent staining reagent is added, and can be an appropriate permissible microbial population.

The CPU board 10 estimates a permissible microbial population N for the complemented number of microorganism. Since the permissible population N appropriately estimates the number of microorganisms, such operation/effects that it is possible to evaluate and apply the ballast water discharge standards (D-2) the same as true evaluation.

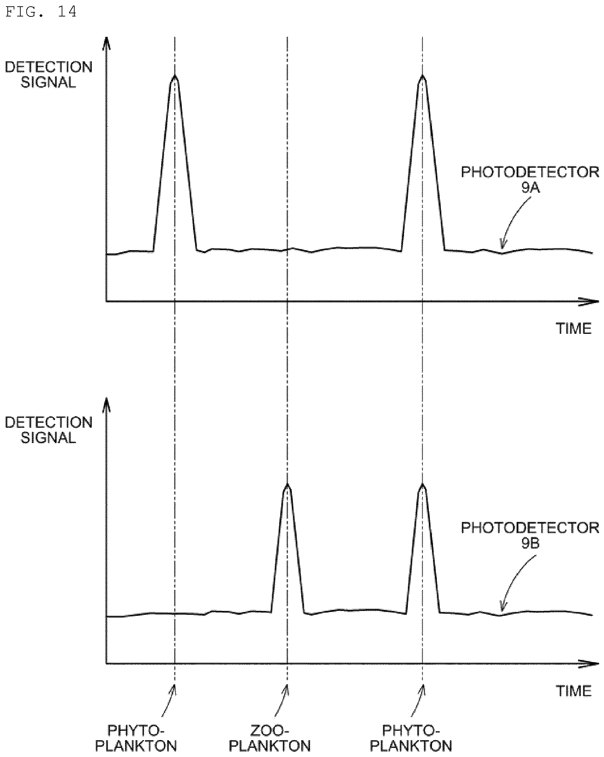

In the case of the modification 2 in Figure 10, while the photodetector 9A mainly detects fluorescence emission only of phytoplankton, the photodetector 9B can detect fluorescence emission of both of the phytoplankton and zooplankton. As shown in Figure 14, by comparing the photodetector 9A and the photodetector 9B, it is possible to, if there is a signal that cannot be detected by the photodetector 9A but detected by the photodetector 9B, estimate the signal as zooplankton. By counting the number of such signals, it becomes possible to grasp the population only of zooplankton.

### [Industrial Applicability]

The present invention can be applied to a microorganism testing apparatus for confirming whether ballast water satisfies a discharge criterion at the time of discharging the ballast water.

### [Reference Signs List]

- 1: testing apparatus
- 2: body portion
- 3: display/operation unit
- 4: sample container
- 5: measuring portion
- 6: stirrer bar
- 7: sample container accommodating portion
- 8: light source portion
- 9: photodetector
- 10: CPU board
- 11: collimator
- 12: band pass filter for excitation light
- 13: band pass filter for fluorescence
- 14: condenser lens
- 15: slit
- 16: relay lens
- 17: long pass filter
- 18: condenser lens
- 20: case
- 21: AC power source
- 22: secondary battery
- 24: AC/DC converter
- 25: RAM
- 26: ROM
- 27: magnetic stirrer
- 28: cooling fan
- 29: external output terminal
- 30: cover
- 31: dichroic mirror
- 32: band pass filter
- 33: long pass filter
- 34: filter wheel
- 35: step motor
- 50: flat plate
- 51: threaded hole
- 53: convex lens

## Claims

1. A microorganism testing apparatus (1) for measuring the number of microorganisms in a sample solution, the microorganism testing apparatus comprising:
a stirring unit (2, 5, 6, 7) for performing stirring of the sample solution and batch-type sample container (4) formed of material transmitting light to which a sample (S) and a fluorescent staining reagent are added; the apparatus further comprising:
an excitation light source (8) provided with a plurality of light sources for emitting excitation light to irradiate a surface of the sample container (4) while the sample solution is being continuously stirred by the stirring unit;
a photodetector (9) for detecting fluorescence light emission from the sample caused by the excitation light from the excitation light source (8);
processing means (10) processing the electrical signals obtained from the light detected by the photodetector (9) to detect and count the number of light emissions, and to calculate the number of microorganisms included in the sample within the sample container (4) from the number of light emissions; and
an operation unit (3) electrically connected to the processing means; wherein the excitation light source (8) comprises two different kinds of excitation light sources (8a, 8b), the excitation light sources being a first light source emitting light with a wavelength region causing phytoplankton to emit chlorophyll fluorescence and a second light source emitting light with a wavelength region causing microorganisms stained by the fluorescent staining reagent to emit fluorescence;
wherein the processing means (10) comprises a calculating unit calculating a number of microorganisms N with respect to a permissible ballast water discharge criterion by determining each of a microbial population n1 acquired from chlorophyll fluorescence emission of the sample without the staining reagent and on excitation by the first light source alone, a microbial population n2 acquired from fluorescence emission by the fluorescent staining reagent after staining of the sample with the staining reagent and on excitation by the second light source alone and a microbial population n3 acquired from both of the chlorophyll fluorescence emission and the fluorescence emission by the fluorescent staining reagent after staining of the sample with the staining reagent and on simultaneous excitation by both of the first and second light sources.

2. The microorganism testing apparatus (1) according to claim 1, wherein the excitation light source (8) is arranged such that excitation light emitted therefrom is caused to be incident orthogonally to the irradiated surface of the sample container (4), and a light receiving surface of the photodetector (9) is arranged such that fluorescence emission is received at an angle orthogonal to the excitation light of the excitation light source (8).

3. The microorganism testing apparatus (1) according to claim 1 or 2, wherein the light sources (8a, 8b) are provided with their own band-pass filter (12A, 12B).

4. The microorganism testing apparatus (1) according to any of claims 1 to 3, wherein a respective photodetector (9A, 9B) is provided for each of the two different kinds of excitation light sources (8a, 8b), and a dichroic mirror (31) is provided for spectrally dispersing the fluorescence due to the excitation light sources to direct dispersed fluorescence to the respective photodetectors.

5. A microorganism testing method for measuring the number of microorganisms in a sample solution, the microorganism testing method comprising:
a stirring process of performing stirring of the sample solution comprising a sample (S) to which has been added a fluorescent staining reagent in a batch-type sample container (4);
an excitation process for irradiating excitation light to a surface of the sample container (4) while continuously stirring the sample solution;
a photodetection process of counting fluorescence emissions from microorganisms caused to emit fluorescence by the excitation process; and
a number-of-microorganisms estimating process of calculating the number of microorganisms included in the sample (S) within the sample container (4) from the number of light emissions detected by the photodetection process; wherein the excitation process causes phytoplankton to be excited by a first light source (8a) emitting light with a wavelength region causing chlorophyll fluorescence emission in a sample without the staining reagent and causes microorganisms stained by the fluorescent staining reagent to be excited by a second light source (8b) emitting light with a wavelength region causing staining reagent fluorescence emission in the sample after staining with the staining reagent, wherein the number-of-microorganisms estimating process comprises calculating a number of microorganisms N with respect to a permissible ballast water discharge criterion after determining each of a microbial population n1 acquired from chlorophyll fluorescence emission in the sample without staining reagent on excitation by the first light source alone, a microbial population n2 acquired from staining reagent fluorescence emission in the sample after staining with the fluorescent staining reagent on excitation by the second light source alone and a microbial population n3 acquired from both chlorophyll fluorescence emission and staining reagent fluorescence emission in the sample after staining with the fluorescent staining reagent on simultaneous excitation by both of the first and second light sources

6. The microorganism testing method according to claim 5, wherein the microorganism estimating process comprises calculating a population of zooplankton by subtracting the microbial population n2 acquired by the fluorescence emission by the fluorescent staining reagent from the microbial population n3 acquired by both of the chlorophyll fluorescence emission and the fluorescence emission by the fluorescent staining reagent.

## Patentansprüche

1. Prüfvorrichtung für Mikroorganismen (1) zum Messen der Anzahl von Mikroorganismen in einer Probenlösung, wobei die Prüfvorrichtung für Mikroorganismen Folgendes umfasst:
eine Rühreinheit (2, 5, 6, 7) zum Durchführen von rühren der Probenlösung und einen chargenweisen Probenbehälter (4) aus lichtdurchlässigen Material, in den eine Probe (S) und ein fluoreszierendes Färbungsreagens gegeben werden; die Vorrichtung umfasst ferner:
eine Anregungslichtquelle (8), die mit mehreren Lichtquellen zum Emittieren von Anregungslicht zum Bestrahlen einer Oberfläche des Probenbehälters (4) versehen ist, während die Probenlösung kontinuierlich von der Rühreinheit gerührt wird;
ein Fotodetektor (9) zum Nachweisen einer fluoreszierenden Lichtemission von der Probe, die durch das Anregungslicht von der Anregungslichtquelle (8) verursacht wird;
Verarbeitungsmittel (10), die die elektrischen Signale, die von dem durch den Fotodetektor (9) nachgewiesenen Licht erhalten werden verarbeiten, um die Anzahl von Lichtemissionen nachzuweisen und zu zählen, und um die Anzahl von Mikroorganismen, die in der Probe in dem Probenbehälter (4) enthalten sind von der Anzahl der Lichtemissionen zu berechnen; und
eine Betriebseinheit (3), die elektrisch mit den Verarbeitungsmitteln verbunden ist; wobei die Anregungslichtquelle (8) zwei verschiedene Arten von Anregungslichtquellen (8a, 8b) umfasst, wobei die Anregungslichtquellen eine erste Lichtquelle, die Licht in einem Wellenlängenbereich emittiert, der Phytoplankton dazu veranlasst, Chlorophyllfluoreszenz zu emittieren und eine zweite Lichtquelle, die Licht einem Wellenlängenbereich emittiert, der mit dem Fluoreszenzfärbungsreagens gefärbte Mikroorganismen dazu veranlasst, Fluoreszenz zu emittieren, sind;
wobei die Verarbeitungsmittel (10) eine Berechnungseinheit umfassen, die eine Anzahl von Mikroorganismen N in Bezug auf ein zulässiges Ballastwasserablasskriterium berechnet, indem jede von einer Mikrobenpopulation n1, die von der Chlorophyllfluoreszenzemission der Probe ohne das Färbungsreagens und bei der Anregung durch die erste Lichtquelle allein erhalten wurde, einer Mikrobenpopulation n2, die von der Fluoreszenzemission durch das Fluoreszenzfärbungsreagens nach der Färbung der Probe mit dem Färbungsreagens und bei der Anregung durch die zweite Lichtquelle allein erhalten wurde, und einer Mikrobenpopulation n3, die von sowohl der Chlorophyllfluoreszenzemission als auch der Fluoreszenzemission durch das Fluoreszenzfärbungsreagens nach der Färbung der Probe mit dem Färbungsreagens und bei der gleichzeitigen Anregung durch sowohl die erste als auch die zweite Lichtquelle erhalten wurde, bestimmt wird.

2. Prüfvorrichtung für Mikroorganismen (1) nach Anspruch 1, wobei die Anregungslichtquelle (8) derart angeordnet ist, dass das davon emittierte Anregungslicht dazu veranlasst wird, orthogonal auf die bestrahlte Oberfläche des Probenbehälters (4) zu treffen, und eine lichtempfangende Oberfläche des Fotodetektors (9) derart angeordnet ist, dass eine Fluoreszenzemission in einem Winkel orthogonal zu dem Anregungslicht der Anregungslichtquelle (8) empfangen wird.

3. Prüfvorrichtung für Mikroorganismen (1) nach Anspruch 1 oder 2, wobei die Lichtquellen (8a, 8b) mit ihrem eigenen Bandpassfilter (12A, 12B) versehen sind.

4. Prüfvorrichtung für Mikroorganismen (1) nach einem der Ansprüche 1 bis 3, wobei ein entsprechender Fotodetektor (9A, 9B) für jede der zwei verschiedenen Arten von Anregungslichtquellen (8a, 8b) bereitgestellt ist, und ein dichroitischer Spiegel (31) zur spektralen Streuung der von den Anregungslichtquellen ausgehenden Fluoreszenz vorgesehen ist, um die gestreute Fluoreszenz zu den entsprechenden Fotodetektoren zu leiten.

5. Prüfverfahren für Mikroorganismen zu messen der Anzahl von Mikroorganismen in einer Probenlösung, wobei das Prüfverfahren für Mikroorganismen Folgendes umfasst:
ein Rührverfahren zum Durchführen von rühren der Probenlösung, umfassend eine Probe (S), zu der ein Fluoreszenzfärbungsreagens in einem chargenweisen Probenbehälter (4) zugegeben wurde;
einen Anregungsprozess zum Bestrahlen einer Oberfläche des Probenbehälters (4) mit Anregungslicht, während die Probenlösung kontinuierlich gerührt wird;
einen Fotodetektionsprozess zum Zählen der Fluoreszenzemissionen von Mikroorganismen, die durch den Anregungsprozess dazu veranlasst werden, Fluoreszenz zu emittieren; und
ein Schätzverfahren für die Anzahl der Mikroorganismen zum Berechnen der Anzahl von Mikroorganismen, die in der Probe (S) in dem Probenbehälter (4) enthalten sind anhand der Anzahl der Lichtemissionen, die durch den Fotodetektionsprozess nachgewiesen werden; wobei der Anregungsprozess bewirkt, dass Phytoplankton durch eine erste Lichtquelle (8a) angeregt wird, die Licht in einem Wellenlängenbereich emittiert, der Chlorophyllfluoreszenzemission in einer Probe ohne das Färbungsreagens verursacht, und bewirkt, dass Mikroorganismen, die durch das Fluoreszenzfärbungsreagens gefärbt wurden, durch eine zweite Lichtquelle (8b) angeregt werden, die Licht in einem Wellenlängenbereich emittiert, der Färbungsreagens-Fluoreszenzemission in der Probe nach dem Färben mit dem Färbungsreagens verursacht,
wobei das Schätzverfahren für die Anzahl der Mikroorganismen das Berechnen einer Anzahl von Mikroorganismen N in Bezug auf ein zulässiges Ballastwasserablasskriterium nach dem Bestimmen jeder von einer Mikrobenpopulation n1, die von der Chlorophyllfluoreszenzemission der Probe ohne das Färbungsreagens bei der Anregung durch die erste Lichtquelle allein erhalten wurde,
einer Mikrobenpopulation n2, die von der Färbungsreagens-Fluoreszenzemission in der Probe nach dem Färben mit dem Fluoreszenzfärbungsreagens bei der Anregung durch die zweiten Lichtquelle allein erhalten wurde,
und einer Mikrobenpopulation n3, die von sowohl der Chlorophyllfluoreszenzemission als auch der Färbungsreagens-Fluoreszenzemission in der Probe nach dem Färben mit dem Fluoreszenzfärbungsreagens bei der gleichzeitigen Anregung durch sowohl die erste als auch die zweite Lichtquelle erhalten wurde, umfasst.

6. Prüfverfahren für Mikroorganismen nach Anspruch 5, wobei das Schätzverfahren für Mikroorganismen das Berechnen einer Population von Zooplankton durch die Subtraktion der Mikrobenpopulation n2, die durch die Fluoreszenzemission von dem Fluoreszenzfärbungsreagens erhalten wurde, von der Mikrobenpopulation n3, die durch sowohl die Chlorophyllfluoreszenzemission als auch die Fluoreszenzemission von dem Fluoreszenzfärbungsreagens erhalten wurde, umfasst.

## Revendications

1. Appareil de test de micro-organismes (1) destiné à mesurer le nombre de micro-organismes dans une solution échantillon, l'appareil de test de micro-organismes comprenant :
une unité d'agitation (2, 5, 6, 7) destinée à réaliser une agitation de la solution échantillon et un récipient d'échantillon de type pour mélange (4) formé de matériau transmettant la lumière auquel un échantillon (S) et un réactif de coloration fluorescent sont ajoutés ; l'appareil comprenant en outre :
une source de lumière d'excitation (8) pourvue d'une pluralité de sources de lumière destinée à émettre une lumière d'excitation pour irradier une surface du récipient d'échantillon (4) alors que la solution échantillon est en train d'être agitée en continu par l'unité d'agitation ;
un photodétecteur (9) destiné à détecter une émission de lumière par fluorescence à partir de l'échantillon provoquée par la lumière d'excitation provenant de la source de lumière d'excitation (8) ;
un moyen de traitement (10) traitant les signaux électriques obtenus à partir de la lumière détectée par le photodétecteur (9) pour détecter et compter le nombre d'émissions de lumière, et pour calculer le nombre de micro-organismes inclus dans l'échantillon à l'intérieur du récipient d'échantillon (4) à partir du nombre d'émissions de lumière ; et
une unité de fonctionnement (3) connectée électriquement au moyen de traitement ; dans lequel la source de lumière d'excitation (8) comprend deux sortes différentes de sources de lumière d'excitation (8a, 8b), les sources de lumière d'excitation étant une première source de lumière émettant de la lumière avec une région de longueur d'onde amenant le phytoplancton à émettre une fluorescence de chlorophylle et une seconde source de lumière émettant de la lumière avec une région de longueur d'onde amenant des micro-organismes colorés par le réactif de coloration fluorescent à émettre une fluorescence ;
dans lequel le moyen de traitement (10) comprend une unité de calcul calculant un nombre de micro-organismes N par rapport à un critère de rejet d'eaux de ballast admissible en déterminant chacune d'une population microbienne n1 acquise à partir de l'émission de fluorescence de chlorophylle de l'échantillon sans le réactif de coloration et lors d'une excitation par la première source de lumière seule, d'une population microbienne n2 acquise à partir de l'émission de fluorescence par le réactif de coloration fluorescent après coloration de l'échantillon avec le réactif de coloration et lors d'une excitation par la seconde source de lumière seule et d'une population microbienne n3 acquise à la fois à partir de l'émission de fluorescence de chlorophylle et l'émission de fluorescence par le réactif de coloration fluorescent après coloration de l'échantillon avec le réactif de coloration et lors d'une excitation simultanée par les première et seconde sources de lumière à la fois.

2. Appareil de test de micro-organismes (1) selon la revendication 1, dans lequel la source de lumière d'excitation (8) est conçue de telle sorte que la lumière d'excitation émise à partir de celui-ci est amenée à être incidente de manière orthogonale à la surface irradiée du récipient d'échantillon (4), et une surface réceptrice de lumière du photodétecteur (9) est conçue de telle sorte que l'émission de fluorescence est reçue à un angle orthogonal à la lumière d'excitation de la source de lumière d'excitation (8).

3. Appareil de test de micro-organismes (1) selon la revendication 1 ou 2, dans lequel les sources de lumière (8a, 8b) sont pourvues de leur propre filtre passe-bande (12A, 12B).

4. Appareil de test de micro-organismes (1) selon l'une quelconque des revendications 1 à 3, dans lequel un photodétecteur (9A, 9B) respectif est prévu pour chacune des deux sortes différentes de sources de lumière d'excitation (8a, 8b), et un miroir dichroïque (31) est prévu pour disperser spectralement la fluorescence due aux sources de lumière d'excitation pour diriger une fluorescence dispersée vers les photodétecteurs respectifs.

5. Procédé de test de micro-organismes destiné à mesurer le nombre de micro-organismes dans une solution échantillon, le procédé de test de micro-organismes comprenant :
un processus d'agitation consistant à réaliser une agitation de la solution échantillon comprenant un échantillon (S) auquel a été ajouté un réactif de coloration fluorescent dans un récipient d'échantillon de type pour mélange (4) ;
un processus d'excitation destiné à irradier une lumière d'excitation sur une surface du récipient d'échantillon (4) tout en agitant de manière continue la solution échantillon ;
un processus de photodétection consistant à compter des émissions de fluorescence à partir de micro-organismes amenés à émettre une fluorescence par le processus d'excitation ; et
un processus d'estimation d'un nombre de micro-organismes consistant à calculer le nombre de micro-organismes inclus dans l'échantillon (S) à l'intérieur du récipient d'échantillon (4) à partir du nombre d'émissions de lumière détectées par le processus de photodétection ; dans lequel le processus d'excitation amène le phytoplancton à être excité par une première source de lumière (8a) émettant de la lumière avec une région de longueur d'onde provoquant une émission de fluorescence de chlorophylle dans un échantillon sans le réactif de coloration et amène des micro-organismes colorés par le réactif de coloration fluorescent à être excités par une seconde source de lumière (8b) émettant de la lumière avec une région de longueur d'onde amenant une émission de fluorescence de réactif de coloration dans l'échantillon après coloration avec le réactif de coloration,
dans lequel le processus d'estimation du nombre de micro-organismes comprend le calcul d'un nombre de micro-organisme N par rapport à un critère de rejet d'eaux de ballast admissible après détermination de chacune d'une population microbienne n1 acquise à partir de l'émission de fluorescence de chlorophylle dans l'échantillon sans réactif de coloration lors de l'excitation par la première source de lumière seule, d'une population microbienne n2 acquise à partir de l'émission de fluorescence de réactif de coloration dans l'échantillon après coloration avec le réactif de coloration fluorescent lors de l'excitation par la seconde source de lumière seule et d'une population microbienne n3 acquise à la fois à partir d'une émission de fluorescence de chlorophylle et une émission de fluorescence du réactif de coloration dans l'échantillon après coloration avec le réactif de coloration fluorescent lors de l'excitation simultanée par les première et seconde sources de lumière à la fois.

6. Procédé de test de micro-organismes selon la revendication 5, dans lequel le processus d'estimation de micro-organismes comprend le calcul d'une population de zooplancton en soustrayant la population microbienne n2 acquise par l'émission de fluorescence par le réactif de coloration fluorescent de la population microbienne n3 acquise par l'émission de fluorescence de chlorophylle et l'émission de fluorescence par le réactif de coloration fluorescent.
